# EUROPEAN PATENT SPECIFICATION

(11) **EP 2 986 720 B1**
(45) Date of publication and mention of the grant of the patent: **06.09.2017**
(21) Application number: 14734916.1
(22) Date of filing: 17.04.2014
(51) Int. Cl.: C12N 9/06, C12N 15/53, A61K 38/44

(54) **URICASE MUTANTS**
URICASE MUTANTEN
MUTANTS DE L'URICASE

(30) Priority: 17.04.2013 IN 1145DE2013
(43) Date of publication of application: 24.02.2016
(73) Proprietor: Council of Scientific & Industrial Research, New Delhi 110 001 (IN)
(72) Inventor: PRASAD, Gandham Satyanarayana, Chandigarh 160036 (IN); YELCHURI, RaviKumar, Chandigarh 160036 (IN)
(74) Representative: Awapatent A/S
(86) International application number: PCT/IN2014/000246
(87) International publication number: WO 2014/170916

(56) References cited:
- WO-A1-2009/120707
- WO-A2-00/08196
- DATABASE UniProt [Online] 16 August 2004 (2004-08-16), "RecName: Full=Uricase; EC=1.7.3.3; AltName: Full=Urate oxidase;", XP002730294, retrieved from EBI accession no. UNIPROT:Q6CNP7 Database accession no. Q6CNP7 -& DUJON BERNARD ET AL: "Genome evolution in yeasts", NATURE (LONDON), vol. 430, no. 6995, 1 July 2004 (2004-07-01), pages 35-44, XP002730312, ISSN: 0028-0836
- KOYAMA YASUJI ET AL: "Cloning, sequence analysis, and expression in Escherichia coli of the gene encoding the Candida utilis urate oxidase (uricase)", JOURNAL OF BIOCHEMISTRY (TOKYO), vol. 120, no. 5, 1996, pages 969-973, XP002729964, ISSN: 0021-924X
- SUZUKI KOJI ET AL: "Molecular cloning and expression of uricase gene from Arthrobacter globiformis in Escherichia coli and characterization of the gene product", JOURNAL OF BIOSCIENCE AND BIOENGINEERING, vol. 98, no. 3, September 2004 (2004-09), pages 153-158, XP4604490, ISSN: 1389-1723 cited in the application
- ITO M ET AL: "IDENTIFICATION OF AN AMINO ACID RESIDUE INVOLVED IN THE SUBSTRATE-BINDING SITE OF RAT LIVER URICASE BY SITE-DIRECTED MUTAGENESIS", BIOCHEMICAL AND BIOPHYSICAL RESEARCH COMMUNICATIONS, ACADEMIC PRESS INC. ORLANDO, FL, US, vol. 187, no. 1, 31 August 1992 (1992-08-31), pages 101-107, XP002923287, ISSN: 0006-291X, DOI: 10.1016/S0006-291X(05)81464-0

### SEQUENCE LISTING

<110> COUNCIL OF SCIENTIFIC AND INDUSTRIAL RESEARCH
   YELCHURI, RAVIKUMAR
   PRASAD, SATYANARAYANA GANDHAM
<120> NOVEL URICASE MUTANT
<130> 1145DEL2013
<160> 14
<170> PatentIn version 3.5
<210> 1
   <211> 308
   <212> PRT
   <213> Kluyveromyces lactis
<220>
   <221> WILDTYPE
   <222> (1)..(308)
<400> 1
   Met Ser Thr Tyr Ser Tyr Leu Ala Asp Ser Thr Tyr Gly Lys Asp Asn
   1               5                   10                  15
   Val Lys Phe Leu Lys Val Lys Lys Ser Lys Thr Asp Pro Lys Val His
               20                  25                  30
   Glu Val Met Glu Cys Asn Val Gln Val Leu Leu Lys Gly Asp Phe Glu
           35                  40                  45
   Val Ser Tyr Thr Lys Ala Asp Asn Thr Pro Ile Val Pro Thr Asp Thr
       50                  55                  60
   Val Lys Asn Thr Ile Leu Val His Ala Arg Lys Phe Asp Thr Trp Pro
   65                  70                  75                  80
   Ile Glu Lys Phe Ala Ala Asn Leu Ala Leu His Phe Thr Gly Lys Tyr
                   85                  90                  95
   Ala His Val Ser Gly Ile Thr Val Lys Ile Ile Gln Asp Arg Trp Ile
               100                 105                 110
   Lys Tyr Asp Val Asp Asn Lys Glu Ser Tyr His Ser Phe Ile His Gln
           115                 120                 125
   Gly Pro Glu Lys Lys Ile Cys Phe Leu Asp Tyr Asp Lys Lys Asn Gly
       130                 135                 140
   Ser Ser Ser Tyr Thr Leu Val Thr Ser Ile Lys Asp Leu Thr Val Leu
   145                 150                 155                 160
   Lys Ser Thr Gly Ser Met Phe Tyr Gly Tyr Asn Val Cys Glu Tyr Thr
                   165                     170                     175
   Thr Leu Lys Pro Thr Thr Asp Arg Ile Leu Ser Thr Asp Ile Phe Ala
               180                 185                 190
   Ser Leu Thr Trp Asn Ser Ala Lys Leu Gly Ser Leu Glu Thr Leu Glu
           195                 200                 205
   Ala Gly Lys Lys Asp Asp Ile Phe Asn Gln Tyr Tyr Ala Ser Ala Arg
       210                 215                 220
   Asp Ile Thr Leu Asp Ile Phe Ala Lys Glu Asn Ser Pro Ser Val Gln
   225                 230                 235                 240
   Ala Thr Met Tyr Asn Met Ala Thr Ala Ile Leu Lys Val Ala Pro Glu
                   245                 250                 255
   Val Glu His Val Ser Tyr Glu Leu Pro Asn Lys His Tyr Ile Leu Phe
               260                 265                 270
   Asp Leu Lys Trp Phe Lys Gly Leu Asp Asn Asn Asn Glu Leu Phe Tyr
           275                 280                 285
   Pro Ser Pro Asp Pro Asn Gly Leu Ile Lys Cys Thr Val Gly Arg Asn
       290                 295                 300
   Gln Ala Lys Leu
   305
<210> 2
   <211> 308
   <212> PRT
   <213> ARTIFICIAL
<220>
   <223> A mutation at the sequence position 62
<220>
   <221> T62S
   <222> (61)..(63)
<400> 2
   Met Ser Thr Tyr Ser Tyr Leu Ala Asp Ser Thr Tyr Gly Lys Asp Asn
   1               5                   10                  15
   Val Lys Phe Leu Lys Val Lys Lys Ser Lys Thr Asp Pro Lys Val His
               20                  25                  30
   Glu Val Met Glu Cys Asn Val Gln Val Leu Leu Lys Gly Asp Phe Glu
           35                  40                  45
   Val Ser Tyr Thr Lys Ala Asp Asn Thr Pro Ile Val Pro Ser Asp Thr
       50                  55                  60
   Val Lys Asn Thr Ile Leu Val His Ala Arg Lys Phe Asp Thr Trp Pro
   65 70 75 80
   Ile Glu Lys Phe Ala Ala Asn Leu Ala Leu His Phe Thr Gly Lys Tyr
   85 90 95
   Ala His Val Ser Gly Ile Thr Val Lys Ile Ile Gln Asp Arg Trp Ile
   100 105 110
   Lys Tyr Asp Val Asp Asn Lys Glu Ser Tyr His Ser Phe Ile His Gln
   115 120 125
   Gly Pro Glu Lys Lys Ile Cys Phe Leu Asp Tyr Asp Lys Lys Asn Gly
   130 135 140
   Ser Ser Ser Tyr Thr Leu Val Thr Ser Ile Lys Asp Leu Thr Val Leu
   145 150 155 160
   Lys Ser Thr Gly Ser Met Phe Tyr Gly Tyr Asn Val Cys Glu Tyr Thr
   165 170 175
   Thr Leu Lys Pro Thr Thr Asp Arg Ile Leu Ser Thr Asp Ile Phe Ala
   180 185 190
   Ser Leu Thr Trp Asn Ser Ala Lys Leu Gly Ser Leu Glu Thr Leu Glu
   195 200 205
   Ala Gly Lys Lys Asp Asp Ile Phe Asn Gln Tyr Tyr Ala Ser Ala Arg
   210 215 220
   Asp Ile Thr Leu Asp Ile Phe Ala Lys Glu Asn Ser Pro Ser Val Gln
   225 230 235 240
   Ala Thr Met Tyr Asn Met Ala Thr Ala Ile Leu Lys Val Ala Pro Glu
   245 250 255
   Val Glu His Val Ser Tyr Glu Leu Pro Asn Lys His Tyr Ile Leu Phe
   260 265 270
   Asp Leu Lys Trp Phe Lys Gly Leu Asp Asn Asn Asn Glu Leu Phe Tyr
   275 280 285
   Pro Ser Pro Asp Pro Asn Gly Leu Ile Lys Cys Thr Val Gly Arg Asn
   290 295 300
   Gln Ala Lys Leu
   305
<210> 3
   <211> 308
   <212> PRT
   <213> ARTIFICIAL
<220>
   <223> A mutation at the postition 167
<220>
   <221> F167W
   <222> (166)..(168)
<400> 3
   Met Ser Thr Tyr Ser Tyr Leu Ala Asp Ser Thr Tyr Gly Lys Asp Asn
   1               5                   10                  15
   Val Lys Phe Leu Lys Val Lys Lys Ser Lys Thr Asp Pro Lys Val His
               20                  25                  30
   Glu Val Met Glu Cys Asn Val Gln Val Leu Leu Lys Gly Asp Phe Glu
           35                  40                  45
   Val Ser Tyr Thr Lys Ala Asp Asn Thr Pro Ile Val Pro Ser Asp Thr
       50                  55                  60
   Val Lys Asn Thr Ile Leu Val His Ala Arg Lys Phe Asp Thr Trp Pro
   65                  70                  75                  80
   Ile Glu Lys Phe Ala Ala Asn Leu Ala Leu His Phe Thr Gly Lys Tyr
                   85                  90                  95
   Ala His Val Ser Gly Ile Thr Val Lys Ile Ile Gln Asp Arg Trp Ile
               100                 105                 110
   Lys Tyr Asp Val Asp Asn Lys Glu Ser Tyr His Ser Phe Ile His Gln
           115                 120                 125
   Gly Pro Glu Lys Lys Ile Cys Phe Leu Asp Tyr Asp Lys Lys Asn Gly
       130                 135                 140
   Ser Ser Ser Tyr Thr Leu Val Thr Ser Ile Lys Asp Leu Thr Val Leu
   145                 150                 155                 160
   Lys Ser Thr Gly Ser Met Trp Tyr Gly Tyr Asn Val Cys Glu Tyr Thr
                   165                 170                 175
   Thr Leu Lys Pro Thr Thr Asp Arg Ile Leu Ser Thr Asp Ile Phe Ala
               180                 185                 190
   Ser Leu Thr Trp Asn Ser Ala Lys Leu Gly Ser Leu Glu Thr Leu Glu
           195                 200                 205
   Ala Gly Lys Lys Asp Asp Ile Phe Asn Gln Tyr Tyr Ala Ser Ala Arg
       210                 215                 220
   Asp Ile Thr Leu Asp Ile Phe Ala Lys Glu Asn Ser Pro Ser Val Gln
   225 230 235 240
   Ala Thr Met Tyr Asn Met Ala Thr Ala Ile Leu Lys Val Ala Pro Glu
   245 250 255
   Val Glu His Val Ser Tyr Glu Leu Pro Asn Lys His Tyr Ile Leu Phe
   260 265 270
   Asp Leu Lys Trp Phe Lys Gly Leu Asp Asn Asn Asn Glu Leu Phe Tyr
   275 280 285
   Pro Ser Pro Asp Pro Asn Gly Leu Ile Lys Cys Thr Val Gly Arg Asn
   290 295 300
   Gln Ala Lys Leu
   305
<210> 4
   <211> 927
   <212> DNA
   <213> Kluyveromyces lactis
<220>
   <221> wildtypeDNA
   <222> (1)..(927)
<400> 4
   atgtcaacct actcatactt agctgattcc acctacggta aggacaatgt caagttttta     60
   aaagtaaaga agtcaaagac tgatccaaaa gttcatgaag ttatggaatg taacgtccaa    120
   gtgttattga agggtgactt tgaagtttct tacacaaagg cagataacac tcctattgtt    180
   ccaactgaca ctgtcaagaa caccatctta gttcatgcta gaaaatttga tacttggcct    240
   attgaaaagt ttgctgctaa tttagcattg catttcactg gcaaatacgc tcatgtttcg    300
   ggtattaccg tcaagatcat tcaagatcgt tggataaagt atgacgttga taacaaagag    360
   agttatcact ccttcattca ccagggtcca gaaaagaaga tttgtttcct agactatgac    420
   aagaagaacg gcagcagcag ttatacttta gtgacatcta ttaaggatct aaccgtgttg    480
   aaatctaccg gatcaatgtt ctacggatac aatgtttgtg aatacaccac tttgaaacca    540
   actactgaca gaatcttgtc aacagatatc tttgcctcat tgacctggaa ttcagcaaaa    600
   cttggttctt tggaaacctt ggaagctggt aagaaggatg acattttcaa ccaatactat    660
   gcatctgctc gtgatataac attggacatc ttcgccaaag agaactctcc atccgtgcaa    720
   gcaacaatgt acaatatggc taccgctatt ttgaaggtgg cacctgaagt tgagcacgtc    780
   agttacgagt tgccaaacaa acattacatc ttgttcgatt tgaaatggtt taagggcttg    840
   gataacaaca atgaattgtt ctacccatct cctgatccaa acgggttgat caagtgcacc    900
   gttggccgta atcaagccaa gctatag                                        927
<210> 5
   <211> 927
   <212> DNA
   <213> ARTIFICIAL
<220>
   <223> artificial sequences
<220>
   <221> T62SDNA
   <222> (183)..(189)
<400> 5
   atgtcaacct actcatactt agctgattcc acctacggta aggacaatgt caagttttta      60
   aaagtaaaga agtcaaagac tgatccaaaa gttcatgaag ttatggaatg taacgtccaa     120
   gtgttattga agggtgactt tgaagtttct tacacaaagg cagataacac tcctattgtt     180
   ccaagcgaca ctgtcaagaa caccatctta gttcatgcta gaaaatttga tacttggcct     240
   attgaaaagt ttgctgctaa tttagcattg catttcactg gcaaatacgc tcatgtttcg     300
   ggtattaccg tcaagatcat tcaagatcgt tggataaagt atgacgttga taacaaagag     360
   agttatcact ccttcattca ccagggtcca gaaaagaaga tttgtttcct agactatgac     420
   aagaagaacg gcagcagcag ttatacttta gtgacatcta ttaaggatct aaccgtgttg     480
   aaatctaccg gatcaatgtt ctacggatac aatgtttgtg aatacaccac tttgaaacca     540
   actactgaca gaatcttgtc aacagatatc tttgcctcat tgacctggaa ttcagcaaaa     600
   cttggttctt tggaaacctt ggaagctggt aagaaggatg acattttcaa ccaatactat     660
   gcatctgctc gtgatataac attggacatc ttcgccaaag agaactctcc atccgtgcaa     720
   gcaacaatgt acaatatggc taccgctatt ttgaaggtgg cacctgaagt tgagcacgtc     780
   agttacgagt tgccaaacaa acattacatc ttgttcgatt tgaaatggtt taagggcttg     840
   gataacaaca atgaattgtt ctacccatct cctgatccaa acgggttgat caagtgcacc     900
   gttggccgta atcaagccaa gctatag                                         927
<210> 6
   <211> 927
   <212> DNA
   <213> ARTIFICIAL
<220>
   <223> artificial sequences
<220>
   <221> F167WDNA
   <222> (498)..(504)
<400> 6
   atgtcaacct actcatactt agctgattcc acctacggta aggacaatgt caagttttta      60
   aaagtaaaga agtcaaagac tgatccaaaa gttcatgaag ttatggaatg taacgtccaa     120
   gtgttattga agggtgactt tgaagtttct tacacaaagg cagataacac tcctattgtt     180
   ccaactgaca ctgtcaagaa caccatctta gttcatgcta gaaaatttga tacttggcct 240
   attgaaaagt ttgctgctaa tttagcattg catttcactg gcaaatacgc tcatgtttcg 300
   ggtattaccg tcaagatcat tcaagatcgt tggataaagt atgacgttga taacaaagag 360
   agttatcact ccttcattca ccagggtcca gaaaagaaga tttgtttcct agactatgac 420
   aagaagaacg gcagcagcag ttatacttta gtgacatcta ttaaggatct aaccgtgttg 480
   aaatctaccg gatcaatgtg gtacggatac aatgtttgtg aatacaccac tttgaaacca 540
   actactgaca gaatcttgtc aacagatatc tttgcctcat tgacctggaa ttcagcaaaa 600
   cttggttctt tggaaacctt ggaagctggt aagaaggatg acattttcaa ccaatactat 660
   gcatctgctc gtgatataac attggacatc ttcgccaaag agaactctcc atccgtgcaa 720
   gcaacaatgt acaatatggc taccgctatt ttgaaggtgg cacctgaagt tgagcacgtc 780
   agttacgagt tgccaaacaa acattacatc ttgttcgatt tgaaatggtt taagggcttg 840
   gataacaaca atgaattgtt ctacccatct cctgatccaa acgggttgat caagtgcacc 900
   gttggccgta atcaagccaa gctatag 927
<210> 7
   <211> 21
   <212> DNA
   <213> Kluyveromyces lactis
<220>
   <221> FORWARDPRIMER
   <222> (1)..(21)
<400> 7
   atgtcaacct actcatactt a 21
<210> 8
   <211> 21
   <212> DNA
   <213> Kluyveromyces lactis
<220>
   <221> REVERSEPRIMER
   <222> (1)..(21)
<400> 8
   ttatagcttg gcttgattac g 21
<210> 9
   <211> 36
   <212> DNA
   <213> ARTIFICIAL
<220>
   <223> artificial sequences
<220>
   <221> MUTANT1FORWARDPRIMER
   <222> (1)..(36)
   <223> MUTANT1FORWARDPRIMER
<400> 9
   cactcctatt gttccaagcg acactgtcaa gaacac 36
<210> 10
   <211> 36
   <212> DNA
   <213> ARTIFICIAL
<220>
   <223> artificial sequences
<220>
   <221> MUTANT1REVERSEPRIMER
   <222> (1)..(36)
   <223> MUTANT1REVERSEPRIMER
<400> 10
   gtgttcttga cagtgtcgct tggaacaata ggagtg 36
<210> 11
   <211> 33
   <212> DNA
   <213> ARTIFICIAL
<220>
   <223> artificial sequences
<220>
   <221> MUTANT2FORWARDPRIMER
   <222> (1)..(33)
   <223> MUTANT2FORWARDPRIMER
<400> 11
   ccggatcaat gtggtacgga tacaatgttt gtg 33
<210> 12
   <211> 33
   <212> DNA
   <213> ARTIFICIAL
<220>
   <223> artificial sequences
<220>
   <221> MUTANT2REVERSEPRIMER
   <222> (1)..(33)
   <223> MUTANT2REVERSEPRIMER
<400> 12
   cacaaacatt gtatccgtac cacattgatc cgg 33
<210> 13
   <211> 6157
   <212> DNA
   <213> Artificial sequence
<220>
   <223> Recombinant Vector having Seq id no :05
<400> 1
<210> 14
   <211> 6157
   <212> DNA
   <213> Artificial sequence
<220>
   <223> Recombinant Vector having seq id no:06
<400> 2

## Description

### FIELD OF THE INVENTION

The present invention relates to novel uricase mutants and their use thereof. More particularly, the present invention relates to uricase mutants, their production and their recombinant peptide sequences and corresponding recombinant nucleotide sequence.

### BACKGROUND OF THE INVENTION

Hyperuricemia is the condition in which the concentration of uric acid in the serum is above the normal range of uric acid levels (2.4 - 6.0 mg/dl for females and 3.4 - 7.0 mg/dl for males)(Chizynski and Rozycka 2005). Even though the cause and effect relationship has not been established (Alderman 2007) for some of the diseases, hyperuricemia is believed to be associated with metabolic syndrome (Choi and Ford 2007) and all the individual components of metabolic syndrome (Choi, Mount et al. 2005; Puig and Martinez 2008; Fraile, Torres et al. 2010)). Causes of hyperuricemia can be due to some disorder in purine degradation pathway or can be due to another disease or some conditions resulting in increased levels of uric acid, like chemotherapy for cancer or kidney diseases. In most of the cases hyperuricemia condition is asymptomatic. Increase in levels of uric acid occur due to over activity of uric acid synthesizing enzymes or defect in uric acid excretion mechanism or both (Yamamoto 2008). Intake of high purine content food (Brule, Sarwar et al. 1992) or the disorders in enzymes of purine degradation pathway like HGPRT, PRPP synthetase, xanthine oxidase increases the levels of uric acid. The excretion of uric acid can be impaired by some antiuricosuric drugs like pyrazinamide, ethambutol, cyclosporine (Scott 1991) or by the improper function of uric acid transporters in kidney. In most of the mixed cases, it is both increase in production of uric acid and decrease in excretion of uric acid through kidney.

Hyperuricemia condition leads to gout and also has number of other significant associations (Feig, Kang et al. 2008). Hyperuricemia is also a risk factor for myocardial infarction and renal diseases (Bos, Koudstaal et al. 2006; Chang, Tung et al. 2010)). It was observed in animals, along with the epidemiological evidence from humans that hyperuricemia was a significant contributor for cardiovascular and vascular diseases (Feig, Kang et al. 2008). Hyperuricemia also plays role in the development of metabolic diseases (Vuorinen-Markkola and Yki-Jarvinen 1994; Cigolini, Targher et al. 1995; Lehto, Niskanen et al. 1998; Fang and Alderman 2000).

Another condition resulted due high urate levels is hyperuricosuria in which the urate crystals are formed in the kidney. The enhanced levels of uric acid in the urine and the defect in its excretion results in the formation of uric acid crystals. This condition may be acute, a complication of tumor lysis syndrome or may be chronic, associated to gout.

Gout is a common metabolic disorder in humans which is characterized by chronic hyperuricemia, deposition of uric acid crystals in the joints and an acute inflammatory arthritis (Choi, Mount et al. 2005). Gout manifests as microscopic and macroscopic deposits of monosodium urate monohydrate crystals tophi which trigger intense acute arthritis with excruciating pain and periarticular inflammation (Mandell 2008). Chronic hyperuricemia in which the serum uric acid levels rises above 9 mg/dl is the most important risk factor for the development of gout. However, only minority of people with hyperuricemia will develop gout (Campion, Glynn et al. 1987; Emmerson 1996; Wortmann 2002). In acute gout, monosodium urate crystals liberated from tissue deposits promote an inflammatory cascade and release of multiple inflammatory cytokines which culminates in neutrophilic inflammation (Liu-Bryan, Scott et al. 2005; Cronstein and Terkeltaub 2006; Martinon, Petrilli et al. 2006).

Humans have uniquely high basal levels of serum uric acid because of missense mutations resulting in a non-functional uricase gene (Yeldandi, Wang et al. 1990; Wu, Muzny et al. 1992). Gout has also been recognized as a complication of cyclosporine therapy among the recipients involved in organ transplantation (Burack, Griffith et al. 1992; Baethge, Work et al. 1993; Ben Hmida, Hachicha et al. 1995). There has been no major change in the treatment of gout since the introduction of colchicum in the sixteenth century" (Hench, Bauer et al. 1935). Treatment has to be done for the acute attack, lowering urate level and lastly prophylaxis to prevent acute gouty attacks. Antihyperuricemic agents are of different types, like xanthine oxidase inhibitors that target uric acid production, uricosurics that enhance the renal uric acid excretion and uricases, the enzymes that degrade uric acid (Bieber and Terkeltaub 2004).

Uric acid is oxidized by uricase to allantoin, which is five times more soluble than uric acid and undergoes efficient renal elimination. Uricase therapies for the gout management could enable to resolute tophi after weeks to months of uricase therapy (Baraf, Matsumoto et al. 2008). Rasburicase, Pegloticase, Uricase-PEG20 are some of the uricase drugs for the treatment of gout. The drugs were effective but their production was difficult, half-life was short and severe allergic reactions were common (Pui, Relling et al. 1997). It was found that uricase administration may cause anaphylaxis, hemolysis and methemoglobinemia in patients with glucose-6-phosphate dehydrogenase deficiency. Later it was found that pegylation reduced serum uric acid levels, was well tolerated and no antibodies were detected (Bomalaski and Clark 2004).

In the process to obtain uricases having better catalytic efficiency, the mutants were created. The present invention is related to two mutant uricases having better catalytic efficiency compared to that of the wild type uricase enzyme.

### SUMMARY OF THE INVENTION

Yet another embodiment of the present invention provides for a method of treating of hyperuricemia, gout and/or reducing serum uric acid levels said method comprising administering to a subject a pharmaceutical composition as described in the present invention. Yet another embodiment of the present invention provides for a pharmaceutical composition as described in the present invention for a preparation of medicament.

Yet another embodiment of the present invention provides for use of a medicament as described in the present invention for the treatment of a disease.

Yet another embodiment of the present invention provides for use of a medicament as described in the present invention for the treatment of hyperuricemia, gout and or reducing serum uric acids levels.

Yet another embodiment of the present invention provides for pharmaceutical composition for the treatment of a metabolic disorder or disease.

Yet another embodiment of the present invention provides for a pharmaceutical composition for the treatment of a metabolic disorder or disease wherein the metabolic disorder or disease is associated with increased or elevated levels of uric acid.

Yet another embodiment of the present invention provides for a pharmaceutical composition for the treatment of metabolic disorder or disease wherein the metabolic disease or disorder includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc.

Another embodiment of the present invention provides for a method of treatment of a metabolic disorder or disease wherein the metabolic disorder or disease is associated with elevated or increased levels of uric acid.

Another embodiment of the present invention provides for a method of treatment of metabolic disorder or disease wherein the metabolic disorder or disease is associated with elevated or increased levels of uric acid includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc..

Another embodiment of the present invention provides for a method of preventing and/or slowing the progression of metabolic disease or metabolic disorder.

Another embodiment of the present disclosure is a method of preventing and/or slowing the progression of metabolic disease or metabolic disorder in particular such metabolic disease or metabolic disorder are caused by increased or elevated uric acid.

Another embodiment of the present invention provides for a method of preventing and/or slowing the progression of metabolic disease or metabolic disorder in particular such metabolic disease or metabolic disorder which includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc.

### BRIEF DESCRIPTION OF THE DRAWINGS

Figure 1: pET 28c vector map
Figure 2: pET28C-K*l*UOM1 recombinant vector comprising SEQ ID No.5
Figure 3: pET28C-K*l*UOM2 recombinant vector comprising SEQ ID No.6

### DETAILED DESCRIPTION OF THE INVENTION

While the invention is susceptible to various modifications and/or alternative processes and/or compositions, specific embodiment thereof has been shown by way of example in the drawings/figures and tables and will be described in detail below. It should be understood, however that it is not intended to limit the invention to the particular processes and/or compositions disclosed, but on the contrary, the invention is to cover all modifications, equivalents, and alternative falling within the scope of the invention as defined by the appended claims.

The graphs, tables, formulas and protocols have been represented where appropriate by conventional representations in the drawings, showing only those specific details that are pertinent to understanding the embodiments of the present invention so as not to obscure the disclosure with details that will be readily apparent to those of ordinary skill in the art having benefit of the description herein.

The following description is of exemplary embodiments only and is not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention.

The terms "comprises", "comprising", or any other variations thereof, are intended to cover a non-exclusive inclusion, such that one or more processes or composition/s or systems or methods proceeded by "comprises... a" does not, without more constraints, preclude the existence of other processes, sub-processes, composition, sub-compositions, minor or major compositions or other elements or other structures or additional processes or compositions or additional elements or additional features or additional characteristics or additional attributes.

### Definitions:

For the purposes of this invention, the following terms will have the meaning as specified therein:
As used herein, the term "subject or patient" when used in the context of the present invention refers to any mammal but not limited to humans.

As used herein, the term "metabolic disease or disorder" when used in the context of the present invention refers to a medical condition caused by disruption of normal metabolism. In the context of the present invention such metabolic disease or disorder includes but not limited to hyperuricemia, gout, Lesch-Nyan syndrome, Cardiovascular disease, Type 2 diabetes, Metabolic syndrome and/or Uric acid stone formation, etc. The metabolic disease or disorder in context of the present invention also includes disease or disorder caused by increased/high/elevated levels of uric acid.

As used herein, the term "uric acid" when used in the context of the present invention refers to is the final oxidation (breakdown) product of purine metabolism and is excreted in urine. Uric acid is oxidized by uricase to allantoin. As used herein, the term "uricase or urate oxidase" when used in the context of the present invention refers to an enzyme responsible for oxidizing uric acid. In the context of the present invention the term uricase is further used for product or enzyme or molecule obtained by the expression of the peptide or protein sequence having SEQ ID No.2 or SEQ ID No. 3 or having nucleotide sequences having SEQ ID No. 5 or SEQ ID No. 6. The term uricase in context of the present invention also refers to mutant strains having accession MTCC No. 5797 and MTCC No. 5798 expressing peptide or protein sequence having SEQ ID No.2 or SEQ ID No. 3 or having nucleotide sequences having SEQ ID No. 5 or SEQ ID No. 6 which are capable of expressing or producing uricase.

The present invention relates to novel mutants of uricase with higher catalytic efficiency than that of the wild type. The present invention also provides for uricase proteins having SEQ ID No. 2 and SEQ ID No. 3. Further the present invention also provides for novel uricase nucleotides having SEQ ID No. 5 and SEQ ID No. 6. An uricase amino acid sequence from K.lactis ATCC8585 showing a glutamic acid at position 53 is disclosed in database UNIPROT accession number :Q6CNP7. The present invention in particular provides that the wild type gene sequence of uricase from *Kluyveromyces lactis* [deposited with International Depository Authority Microbial Type Culture Collection (MTCC), Chandigarh, India having the accession number MTCC No.458] which encodes an uricase with a Tryptophan at position 62 and Phenylalanine at position 167 has been replaced by Serine and Tryptophan respectively (T62S and/or F167W). It has been found in the present invention that replacement of amino acids at the positions 62 and 167 of *Kluyveromyces lactis* results in unexpected increase in the uricase activity.

The invention also provides a method of preparing the novel uricase mutant strains. Further the invention also provides recombinant vectors capable of carrying and expressing nucleotides and proteins of the present invention. The mutant uricases strains of the present invention are prepared by conventional recombinant DNA techniques e.g. those described in "Sambrook et al., Molecular Cloning: A Laboratory Manual" (III^{rd} Ed., Cold Spring Harbor Press, 2001) among several other manuals/compendia of protocols. and the techniques of protein purification and characterization, in particular the various chromatographic methods employed conventionally for purification and downstream processing recombinant proteins and enzymes viz, ion-exchange and gel filtration chromatography, and affinity chromatographic techniques well known in the field of protein biochemistry.

The invention further provides a method for the preparation of mutant uricases possessing better catalytic efficiency, said method comprising steps of: (a) preparing DNA encoding wild type uricase by conventional biochemical or chemical methods or appropriate combinations thereof, to express a translational product, which is a wild type uricase; (b) creating mutations in the said wild type uricase gene by genetic engineering means to produce a translational product which is mutant uricases; (c) introducing the mutant DNA obtained in step (b) in usual manner into a vector such as plasmid or cosmid or bacteriophage for transformation of appropriate host, selected from the group comprising *E. coli,* yeast or mammals; (c) the resulting strains can be screened for the target transformant, carrying the recombinant vector having the mutant DNA sequence and having the ability to produce the mutant uricases; and (d) the mutant uricases strains of the present invention can be obtained by culturing the transformants; expression of the protein from certain promoters can be elevated in the presence of certain inducers; disrupting the cells by conventional methods comprising grinding, enzyme treatment, ultrasonication; and then purifying the mutant uricases using conventional procedures comprising centrifugation, affinity chromatography, gel filtration or combinations thereof.

In another aspect the present invention provides novel primers having SEQ ID No. 9, SEQ ID No.10, SEQ ID No.11 and SEQ ID No.12 which are used for amplification of SEQ ID No. 5 and SEQ ID No. 6. The primers having SEQ ID No. 9 and SEQ ID No.10 are used as forward and reverse primers for SEQ ID No. 5 (PCR amplification). The primers having SEQ ID No. 11 and SEQ ID No.12 are used as forward and reverse primers for SEQ ID No. 6 (PCR amplification). In another aspect of the present invention provides for recombinant vector consisting of peptide sequence having SEQ ID No. 2. In yet another aspect of the present invention provides for recombinant vector consisting of peptide sequence having SEQ ID No. 3.

In another aspect of the present invention the SEQ ID No. 2 is a peptide or protein sequence wherein the amino acid tryptophan at position 62 has been replaced by amino acid serine (T62S). The present invention also provides a peptide or protein sequence having SEQ ID No. 3 wherein the amino acid phenylalanine at position 167 has been replaced by amino acid tryptophan (F167W).

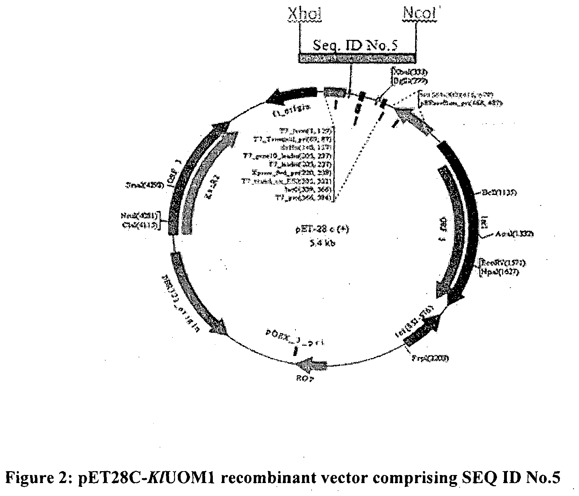

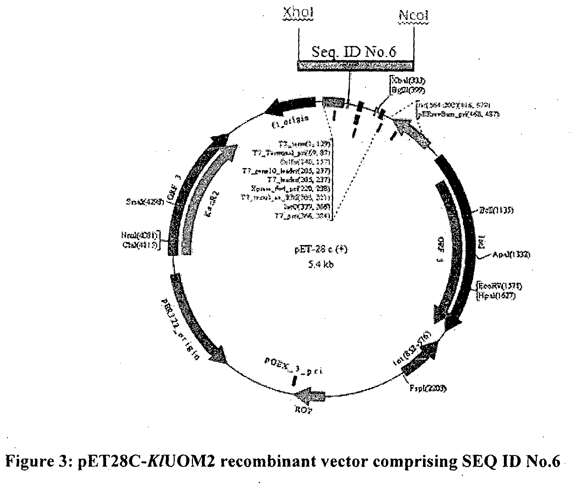

In another aspect the present invention provides for a recombinant vector comprising SEQ ID No. 5 **(****Figure 2****).** In another aspect the present invention provides for a recombinant vector comprising SEQ ID No. 5 **(****Figure 3****).**

In one aspect the present invention provides a pharmaceutical composition comprising an uricase peptide or protein having SEQ ID No.2 and/or SEQ ID No.3 along with pharmaceutically acceptable carrier. In another aspect the present invention also provides for a pharmaceutical composition comprising peptide having SEQ ID No. 5 and/or SEQ ID No.6 along with pharmaceutically acceptable carrier.

In another aspect the present invention provides for a pharmaceutical composition produced, manufactured or prepared by a host cell comprising a recombinant vector capable of expressing sequences selected group consisting of SEQ ID No.2 or SEQ ID No.3 or SEQ ID No.5 or SEQ ID No.6.

The Uricases from *Aspergillus flavus* [pH 8.0, (Li, Chen et al. 2006)], chimeric pig-baboon uricase (pH 9.2, US8293228) are being used as drugs and uricase of *Candida utilis* (pH 8.5, EP 2271750) is under phase III clinical trials. These enzymes retain only partial activity at biological pH when used as therapeutic agents as their optimum pH is around 8 to 8.5. The optimum pH of uricase of the present invention has been found to be close to the biological pH. This property is of significance since it has been found that the enzyme activity is retained almost in its entirety (close to 100%) when administered as drug. Some other uricases reported in the literature were shown to have optimum pH of 7.0 (Suzuki, Sakasegawa et al. 2004). However the stability of enzyme is in the pH range of 8.5 to 11 indicating that this enzyme will have low activity at biological pH. The uricase of *Candida utilis* was reported to have better catalytic efficiency than other uricases reported so far. However, the catalytic efficiency of uricase mutant strains of this patent invention has been found to be higher than that of the uricase of *Candida utilis* (2 to 3 fold higher) when examined under similar experimental conditions in our laboratory along with the other enzymes available in the market (the values are not reported in any literature) **(Table 1).**

K_{M} (Michelis and Menten constant) indicates the affinity of the enzyme towards the substrate molecules. Affinity is inversely proportional to the K_{M} value, i.e. lower K_{M} value indicates better affinity of the enzyme towards the substrate. Kcat represents catalytic turnover number of the enzyme, i.e. number of substrate molecules converted per unit time, which is expressed as k_{cat} (min⁻¹) in the above table. Higher Kcat value indicates the enhanced ability of the enzyme to convert the substrate molecules. The overall catalytic efficiency of the enzymes is expressed as k_{cat}/K_{M} (µM⁻¹ min⁻¹

Table 1 shows the comparative enzyme kinetic study of uricases of this invention and the already available ones. The kinetics of uricases of *A.* flavus, C. *utilis* and *A. globiformis* along with the uricases of this invention were studied under similar laboratory conditions. The mutants SEQ ID No.2 and SEQ ID No.3 of the present invention showed better enzyme efficiency (k_{cat}/K_{M} = 0.88 and 0.516 respectively) than all the wild type uricase of other species.

**Table 1: Comparative enzyme kinetic study of uricases**

| **S. No.** | **Organism** | **K_{M} (µM)** | **k_{cat} (min⁻¹)** | **k_{cat}/K_{M} (µM⁻¹ min⁻¹)** |
|---|---|---|---|---|
| 1 | *A. flavus* | 96.2 | 4.3 | 0.044 |
| **2** | ***C. utilis*** | **38.5 ± 5.16** | **12.16** | **0.315** |
| 3 | *A. globiformis* | 215±2.16 | 0.87 | 0.004 |
| 4 | **K*l*UOX (wild type) (SEQ ID NO.1)** | **40.8** | **12.16** | **0.29** |
| 5 | **KI T62S (SEQ ID NO.2)** | **16.2** | **14.32** | **0.88** |
| 6 | **KI F167W (SEQ ID NO.3)** | **28.07** | **14.5** | **0.516** |

The present invention also provides for pharmaceutical composition of the uricase as described in the present invention. The pharmaceutical composition of the present invention is intended for parenteral and oral administration. Preferably, the pharmaceutical composition described as herein in the present invention can be administered parenterally for example, intravenously, subcutaneously, intradermally or intramuscularly. The present invention also provides for agents which function as "pharmaceutically acceptable carrier or pharmaceutically acceptable excipient", wherein the term "pharmaceutically acceptable excipient or pharmaceutically acceptable excipient" means a pharmaceutically acceptable excipient or carrier, solution or additive to enable the delivery, dissolution or suspension of the pharmaceutical active ingredient as herein as described in the present invention. The active ingredient as described in the context of the present invention is the uricase protein produced by peptide or nucleotide sequence having SEQ ID No. 2 or SEQ ID No.3 or SEQ ID No. 5 or SEQ ID No. 6. The pharmaceutical composition of the present invention may also contain pharmaceutically accepted auxiliary substances as required to approximate physiological conditions, such as pH adjusting and buffering agents, tonicity adjusting agents, wetting agents and the like. The pharmaceutical composition of the present invention may also contain pharmaceutically acceptable carriers, for example adjuvants, etc. The pharmaceutical composition of the present invention may also comprise of uricase conjugated with polymer or biopolymer, wherein the polymers can be common or commercially known or used polymers particularly pharmaceutical administered compositions, for e.g. PEG or its derivatives, dextran etc. The pharmaceutical compositions of this invention may also be administered in any convenient form, for example tablet, capsule, injection, granule or powder form. The pharmaceutical composition of the present invention is prepared in such a manner so that it retains the uticolytic activity of the uricase to bring maximum benefit to the patient on administration.

The present invention also provides a method of treating, preventing and/or slowing the progression of metabolic disease or metabolic disorder. Another aspect of the present invention provides for a method of treating, preventing and/or slowing the progression of metabolic disease or metabolic disorder in particular such metabolic disease or metabolic disorder are caused by increased or elevated uric acid. The various diseases or disorders associated with increased or elevated levels of uric acid includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc. The present invention also provides for method of treating, preventing or slowing the progression of disease or disorder caused by increased or elevated uric acid by administering a pharmaceutical composition as described in the present invention. Herein is described a method of treating of hyperuricemia, gout and/or reducing serum uric acid levels said method comprising administering to a subject a pharmaceutical composition as described in the present invention. Yet another embodiment of the present invention provides for a pharmaceutical composition as described in the present invention for a preparation of medicament.

Yet another embodiment of the present invention provides for use of a medicament as described in the present invention for the treatment of a disease.

Yet another embodiment of the present invention provides a medicament as described in the present invention for the treatment of hyperuricemia, gout and or reducing serum uric acids levels.

Yet another embodiment of the present invention provides for pharmaceutical composition for the treatment of a metabolic disorder or disease.

Yet another embodiment of the present invention provides for a pharmaceutical composition for the treatment of a metabolic disorder or disease wherein the metabolic disorder or disease is associated with increased or elevated levels of uric acid.

Yet another embodiment of the present invention provides for a pharmaceutical composition for the treatment of metabolic disorder or disease wherein the metabolic disease or disorder includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc.

Another embodiment of the present invention provides for a method of treatment of a metabolic disorder or disease wherein the metabolic disorder or disease is associated with elevated or increased levels of uric acid.

Another embodiment of the present invention provides for a method of treatment of metabolic disorder or disease wherein the metabolic disorder or disease is associated with elevated or increased levels of uric acid includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc..

Another embodiment of the present invention provides for a method of preventing and/or slowing the progression of metabolic disease or metabolic disorder.

Another embodiment of the present invention provides for a method of preventing and/or slowing the progression of metabolic disease or metabolic disorder in particular such metabolic disease or metabolic disorder are caused by increased or elevated uric acid.

Another embodiment of the present invention provides for a method of preventing and/or slowing the progression of metabolic disease or metabolic disorder in particular such metabolic disease or metabolic disorder which includes but not limited to, hyperuricemia, gout, Lesch-Nyan syndrome, Cardiovascular disease, Type 2 diabetes, Metabolic syndrome and/or Uric acid stone formation, Tumor lysis syndrome, osteogenesis imperfecta etc.

The main advantages of the present invention are:
1. The present invention provides mutant uricase having higher activity.
2. The optimum pH of these mutants is close to the biological pH 7 which helps the enzyme to retain almost entire activity (close to 100%) when administered as drug.
3. The catalytic efficiency of mutants of this disclosure are found to be higher than that of the other modified uricases (2 to 3 times fold higher)
4. The uricase mutants of the present invention can be used in clinical diagnosis kits to measure the serum uric acid levels and as a potential drug for hyperuricemia

The present disclosure also provides for use of uricase mutant strains or peptide sequences or protein sequences as described in the present invention for clinical diagnosis kits to measure the serum uric acid levels and as a therapeutic drug for the treatment of hyperuricemia.

The present disclosure also provides use of uricase mutant strains or peptide sequences or protein sequences as described in the present invention for diagnosis of metabolic disease or disorder. In another aspect the present disclosure also provides for use of uricase mutant strains or peptide sequences or protein sequences as described in the present invention for diagnosis of metabolic disease or disorder caused by elevated or increased uric acid in a subject. Another aspect of the disclosure provides for a method of determining or diagnosis of a metabolic disorder or disease in particular such metabolic disorder or disease caused by elevated or increased uric acid in a subject who is risk of such a metabolic disease or disorder as described in the present invention.

The following description is of exemplary embodiments only and is not intended to limit the scope, applicability or configuration to the invention in any way, Rather, the following description provides a convenient illustration for implementing exemplary embodiments of the invention various changes to the described embodiments may be made in the functions and arrangement of the elements described without departing from the scope of the invention.

### EXAMPLES

### Example 1: Experimental Methods used:

**Recombinant DNA methods:** In general, the methods and techniques of rDNA well known in the area of molecular biology were utilized. These are readily available from standard texts and protocol pertaining field of the art, for example, Sambrook et al., Molecular Cloning: A Laboratory Manual" (IIIrd Ed., Cold Spring Harbor Press, 2001; McPherson, M. J., Quirk P., and Taylor, G. R., [Ed.] PCR: A Practical Approach. IRL press, oxford., 1991). However, pertinent in the context of experiments describing the present invention, particularly where modifications were introduced to established procedures, are indicated in the examples wherever relevant.

**SDS-PAGE analysis of proteins:** SDS-PAGE is carried out, essentially according to Laemmli, U.K., 1970, Nature 227:680 with minor modifications, as needed. Briefly, protein samples are prepared by mixing with an equal volume of the 2X sample buffer (0.1 M TrisCl, pH 6.8; 6 % SDS; 30 % glycerol; 15 % beta-mercaptoethanol and 0.01% Bromophenol Blue dye). Prior to loading onto the gel, the samples are heated in a boiling water bath for 5 min. The discontinuous gel system usually has 5 % (acrylamide conc.) in the stacking and 12 % in the resolving gel. Electrophoresis is carried out using Laemmli buffer at a constant current of 15 mA first, till the samples enter the gel and then 30 mA till the completion. On completion of electrophoresis, gel is immersed in 0.1% coomasie Blue R250 in methanol: acetic acid: water (4:1:5) with gentle shaking and is then destained in destaining solution (20 % methanol and 10 % glacial acetic acid) till the background becomes clear.

**Enzymatic assay of uricase:** Substrate mixture contains
a. 2.2 ml of 2 mM Uric acid
b. 0.15 ml of 60 mM 4-Aminoantipyrine (4-AAP)
c. 0.15 ml of 1.5% Phenol
d. 0.15 ml of 20 U/ml Horseradish peroxidase (HRP)
e. 0.1 M respective buffer to make up to 3 ml
   a. Uricase added to the above mixture and incubated at 25°C for 20 min. The colour is measured at 505 nm (Chen, Wang et al. 2008).
   b. The reaction mix was set for 300 µl rather than 3 ml for convenient measuring in BiotekPowerwave X Elisa plate reader. The amounts of the solutions added in the assay mix were calculated accordingly for 300 µl.

Uric acid dissolves in alkaline pH and all the reagents kept at room temperature except HRP which was kept at 4°C.One unit of enzyme will convert 1.0 µmole of uric acid to allantoin per min at its optimum pH and temperature.

### Example 2: Preparation of wild type uricase (SEQ ID No. 4)

The uricase gene having SEQ ID No.4 was isolated from the genomic DNA of *Kluyveromyces lactis* (MTCC No. 458) using the specific primers having SEQ ID No. 7 and SEQ ID No. 8 **(Table 2).**

**Table 2: Specific primers for the SEQ ID NO.4**

| | | |
|---|---|---|
| **SEQ ID No. 7** | *Kl*UOX F | ATGTCAACCTACTCATACTTA |
| **SEQ ID No. 8** | *Kl*UOX R | TTATAGCTTGGCTTGATTACG |

The uricase gene was amplified using the specific primers in PCR program. The reaction mix and the PCR program are mentioned below. The NEB (New England Biolabs, UK) reagents were used for the preparation of reaction mix.

| | |
|---|---|
| Enzyme buffer (10X) | 5.0 µl |
| Template DNA | 5.0 ng (genomic DNA) |
| Forward primer | 1 µM |
| Reverse primer | 1 µM |
| dNTPs | 400 µM |
| DNA polymerase enzyme | 1 U/ 50 µ |

Reaction volume was made to 50.0 µl by autoclaved DDW. The PCR reaction was carried out as described below

| | |
|---|---|
| Step 1. Melting temperature | 95°C for 5 min |
| Step 2. Melting temperature | 95°C for 1 min |
| Step 3. Annealing temperature | 55°Cfor 1 min |
| Step 4. Elongation temperature | 72°C for 1 min |
| Go to step 2 and repeat 30 cycles | |
| Step 5. Elongation temperature | 72°C for 1 min |
| Step 6.Hold at 4°C. | |

The amplified product was purified using QIAquick^{®} Gel Extraction kit (Qiagen, Germany).

### Example 2: Preparation of mutant uricase genes having SEQ ID No. 5 and SEQ ID No. 6

The wild type uricase gene was used as template to prepare mutants in the PCR based method using overlapping primers as in those described in "Sambrook et al., Molecular Cloning: A Laboratory Manual" (III^{rd} Ed., Cold Spring Harbor Press, 2001) using the specific overlapping primers, mentioned in **Table 3.**

**Table 3: Specific primers for the SEQ ID No.4, SEQ ID No.5 & SEQ ID No.6.**

| | | |
|---|---|---|
| **SEQ ID No. 7** | KlUOX F | ATGTCAACCTACTCATACTTA |
| **SEQ ID No. 8** | KlUOX R | TTATAGCTTGGCTTGATTACG |
| **SEQ ID No. 9** | Kl M1 F | CACTCCTATTGTTCCAA**GCG**ACACTGTCAAGAACAC |
| **SEQ ID No. 10** | Kl M1 R | GTGTTCTTGACAGTGT**CGC**TTGGAACAATAGGAGTG |
| **SEQ ID No. 11** | Kl M2 F | CCGGATCAATG**TGG**TACGGATACAATGTTTGTG |
| **SEQ ID NO. 12** | Kl M2 R | CACAAACATTGTATCCGTA**CCA**CATTGATCCGG |
| **SEQ ID NO. 13** | Kl CT F | CATGCCATGGGGATGTCAACCTACTCATACTTA |
| **SEQ ID NO. 14** | Kl CT R | CCGCTCGAGTAGCTTGGCTTGATTACG |

The uricase gene was amplified using the specific primers in PCR program. The reaction mix and the PCR program are mentioned below. The NEB (New England Biolabs, UK) reagents were used for the preparation of reaction mix.

| | |
|---|---|
| Enzyme buffer (10X) | 5.0 µl |
| Template DNA | 1.0 ng (wild type uricase gene) |
| Forward primer | 1 µM |
| Reverse primer | 1 µM |
| dNTPs | 400 µM |
| DNA polymerase enzyme | 1 U/ 50 µl |

Reaction volume was made to 50.0 µl by autoclaved DDW. The PCR reaction was carried out as described below

| | |
|---|---|
| Step 1. Melting temperature | 95°C for 5 min |
| Step 2. Melting temperature | 95°C for 1 min |
| Step 3. Annealing temperature | 55°Cfor 1 min |
| Step 4. Elongation temperature | 72°C for 1 min |
| Go to step 2 and repeat 30 cycles | |
| Step 5. Elongation temperature | 72°C for 1 min |
| Step 6.Hold at 4°C. | |

The amplified product was purified using QIAquick^{®} Gel Extraction kit (Qiagen, Germany).

### Example 3: Preparation of recombinant plasmid with mutant uricases SEQ ID No. 5 & SEQ ID No.6

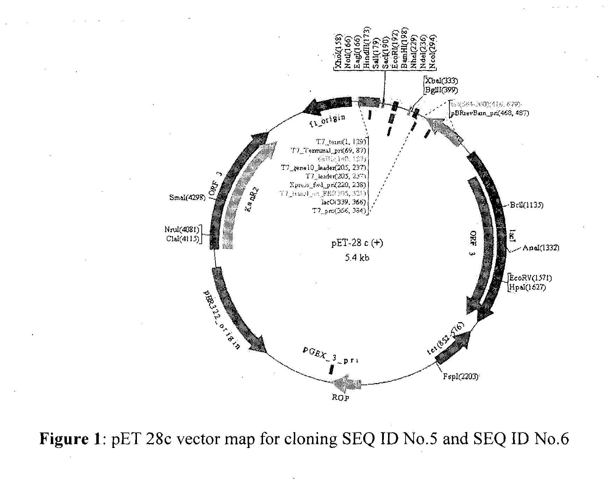

The restriction sites of *Nco*I and *Xho*I were inserted in the forward and reverse primers respectively (Restriction sites of *Nco*I and *Xho*I were inserted in primers with SEQ ID no. 7 and 8 and primers with restriction sites are provided in Table 3 with SEQ ID no. 13 and 14 respectively). Cloning the gene at these enzymes sites in the vector pET28c **(****Figure 1****)** would allow the gene to express into a protein under the inducible T7 promoter with 6x His-tag at the C-terminal end. The uricase gene was amplified with these primers by standard PCR protocol mentioned above and the amplified products were purified using the gel purification kit as mentioned earlier. The amplified PCR products and pET 28c vector digested with the enzymes *Nco*I and *Xho*I (New England Biolabs, USA) for 3 hrs at 37 °C and digested products were gel purified. Insert and vector were added in the ratio 3:1 and ligated using T4 DNA ligase (New England Biolabs, USA) for 16hrs at 16 °C. The resulting plasmids were designated as pET28C-*Kl*UOM1 (mutant 1, SEQ ID No.5; **Figure 2****)** and pET28C-*Kl*UOM2 (mutant 2, SEQ ID No.6; **Figure 3****).**

### Example 4: Expression and purification of the mutant uricases from E. coli

The recombinant plasmids pET28C-*Kl*UOM1 (mutant 1, SEQ ID No.5) and pET28C-*Kl*UOM2 (mutant 2, SEQ ID No.6) were then transformed by heat shock method into the chemically competent *E. coli* strain BL21 (DE3) cells. Randomly selected colonies were inoculated in LB broth supplemented with 50 µg/ml kanamycin antibiotics (LB+Kan) and incubated overnightat 37°C. The plasmids were isolated from the broth culture of clones using commercial plasmid extraction kit (Qiagen, Germany). The presence of insert was confirmed in randomly selected colonies by both double digestion and PCR amplification of the insert. The positive clones carrying the recombinant plasmidspET28C-*Kl*UOM1 (mutant 1 SEQ ID No.5) and pET28C-*Kl*UOM2 (mutant 2 SEQ ID No.6) were in 20 ml LB+Kan broth and incubated at 37°C overnight.A 10 ml of overnight grown each seed culture were inoculated into 1,000 ml of fresh LB+Kan broth and was allowed to grow further at 37°C on a rotary shaking (200 rpm) incubator till it reached 0.6 - 0.8 OD at 600 nm. The protein expression was induced by adding 1 mM IPTG and the cells were allowed to grow further for 3 hr at 37°C in a rotary shaking (200 rpm) incubator. After 3 hr of induction, the cells were harvested by centrifuging the culture for 15 min at 5,000 rpm at RT. The bacterial cell pellets wereresuspended uniformly in lysis buffer (5 ml/lgm of wet weight of cell pellet) using vortex or pipetteman. Lysis buffer constitutes 50 mMTris-HCL pH 8.5, 150 mMNaCl, 10 mM imidazole, 1 mM DTT, 1 mg/ml lysozyme and 0.2 ml of protease cocktail Inhibitor (Sigma, USA). The resuspended cells were lysed by sonication (Sonics, USA); the sample was centrifuged and supernatant was taken in fresh tube. The Nickel-Nitrilotriacetic acid (Ni-NTA) column (Qiagen, Germany) was pre-equilibrated with 10 column volumes of equilibration buffer (50 mMTris-HCL pH 8.5, 150 mMNaCl, 10 mM Imidazole, 1 mM DTT).After equilibrating, supernatant of protein preparation was added to the Ni-NTA column and allowed to pass. After passing the supernatant the column was washed with 25 column volumes of wash buffer (50 mMTris-HCl pH 8.5, 150 mMNaCl, 1 mM DTT and 20 mM imidazole). After wash, the bound protein was eluted with elution buffer (50 mMTris-HCl pH 8.5, 150 mMNaCl, 1 mM DTT and 200 mM imidazole). The elutions were examined using 12 % SDS-PAGE. The elutions with visible protein band were pooled and dialyzed overnight against the dialysis buffer (50 mMTris-HCl pH 8.5, 150 mMNaCl, 1 mM DTT) in cold room at 4°C.After Ni-NTA purification of recombinant proteins, a fewer contaminant protein could be removed by gel-filtration chromatography. We used Sephacryl S-200 column for protein purification (GE healthcare) installed in Biorad FPLC system. The technique was also used to analyze the hydrodynamic volume of proteins. Recombinant proteins were dialyzed against appropriate buffers using the Small Wonder-Lyzer dialysis bag (Excellion Innovations and Inventions Inc., US patent no. 6368509) having a 10 kDa molecular weight cut off (cellulose) membrane.

### Example 5: Characterization of wild type and mutant uricases (mutant 1 & mutant 2)

### 1. Determination of optimum pH of uricases

To measure optimum pH of uricases the present invention used different pH buffers ranging from 3 to 12 and the uricases activity were assayed in each of these buffers under same conditions. Different buffers were used to provide desired pH for the assay. Citrate buffer for the pH 3.0, 4.0, 5.0, potassium dihydrogen phosphate buffer for pH 6.0, 7.0, 8.0, sodium tetra borate buffer for pH 8.5, 9.0, 10.0, sodium bicarbonate buffer for pH 11.0 and potassium chloride buffer for pH 12.0 were used. All these buffers were of 0.1 M concentration and adjusted to the final pH with respective bases or acids. The uric acid was dissolved in these buffers according to the final pH of the reaction. All other reagents were added in each reaction and finally equal amount of purified enzyme was added in all the wells to make up the volume of the assay mix to 300 µl. Incubated the mix at 25°C for 20 min and the reaction was stopped using KOH. Immediately the intensity of the color was measured in ELISA plate reader at 505 nm. The optimum pH for the wild type as well as both mutants was found to be pH 7 (Table 4), which is close to the biological pH.

**Table 4: Optimum pH of the uricases of this invention**

| **S. No** | **Name of Mutant** | **Optimum pH** |
|---|---|---|
| 1 | **Wild type** | **7.0** |
| 2 | **Mutant 1** | **7.0** |
| 3 | **Mutant 2** | **7.0** |

### 2. Kinetic studies of uricases

The kinetics of the uricases was studied to evaluate the efficiency of the enzymes. The kinetic parameters of the uricases of all the species were determined at their respective optimum pH and optimum temperatures. The substrate (uric acid) was added in increasing concentrations ranging from 10 to 600 µM (10, 20, 40, 80, 100, 200, 300, 400, 500, and 600) in triplicates. The assay reagents for the kinetic studies were added in 96 well plates in triplicates in the buffer of pH 7.0 and incubated at 35°C. Equal amounts of enzyme were added and immediately readings were taken at 505 nm. Around 30 readings were taken at the interval of 1min. The readings at a particular time point were taken for further steady state kinetic study of uricases. The K_{M} and Vₘₐₓ values were determined from Michaelis-Menten saturation curve by the non-linear curve fit (ORIGIN 7.5). kcat and kcat/K_{M} values were also calculated (table 5).

**Table 5: Kinetic properties of the uricases of this invention**

| **S. No** | **Name of Mutant** | **Km (µM)** | **Kcat (min-1)** | **Kcat/Km (µM-1 min-1)** |
|---|---|---|---|---|
| 1 | Wild type (SEQ ID No. 1) | 40.8 | 12.16 | 0.29 |
| 2 | Mutant 1 (SEQ ID No. 2) | 16.2 | 14.32 | 0.88 |
| 3 | Mutant 2 (SEQ ID No. 3) | 28.07 | 14.5 | 0.516 |

**Table 6: E. coli strains carrying mutant uricase genes were deposited in International Depository Authority -Microbial Type Culture Collection (IDA-MTCC, India).**

| **S. No** | **Name of Mutant** | **MTCC No.** |
|---|---|---|
| 1 | Wild type ((SEQ ID NO 1) | MTCC 5796 |
| 2 | Mutant 1 (SEQ ID NO 2) | MTCC 5797 |
| 3 | Mutant 2 (SEQ ID NO 3) | MTCC 5798 |

## Claims

1. Uricase mutant strains selected from deposits having accession numbers MTCC No. 5797 and MTCC No. 5798.

2. The uricase mutant strains as claimed in claim 1 expressing proteins with SEQ ID No.: 2 or SEQ ID No.:3, wherein the proteins are encoded by nucleotides having a SEQ ID No: 5 or SEQ ID No.: 6.

3. A recombinant vector capable of expressing proteins having sequences SEQ ID No. 2 and SEQ ID No. 3.

4. The recombinant vector as claimed in claim 3, comprising nucleotide having sequences SEQ ID No. 5 and SEQ ID No. 6.

5. A method for preparation of mutant strains of uricase having accession numbers MTCC No. 5797 & MTCC No.5798, said method comprising steps of:
(a) isolating a nucleotide of interest having SEQ ID No. 4 using primers;
(b) creating mutation in SEQ ID No.4 at position corresponding to position 62 and /or 167 in the protein sequence to obtain recombinant sequences;
(c) cloning of the recombinant nucleotides obtained in step (b) in a vector construct to obtain a recombinant vector;
(d) transforming the recombinant vector obtained in step (d) in a suitable host for expression of the recombinant sequences; and
(e) obtaining the mutant strains of uricase.

6. The method as claimed in claim 5, wherein the recombinant nucleotides have SEQ ID No: 5 and SEQ ID No: 6 and said recombinant nucleotides encode recombinant proteins having SEQ ID No.: 2 and SEQ ID No.: 3.

7. The method as claimed in claim 6, wherein the recombinant proteins are uricase proteins

8. The method as claimed in claim 5, wherein the primers are represented by SEQ ID No.: 9, SEQ ID No.: 10, SEQ ID No.: 11 and SEQ ID No.: 12, wherein SEQ ID No.: 9 and SEQ ID No.: 10 are forward and reverse primers for nucleotide with SEQ ID No.: 5 and SEQ ID No.: 11 and SEQ ID No.: 12 are forward and reverse primers for nucleotide with SEQ ID No: 6.

9. A pharmaceutical composition comprising an uricase as claimed in claims 1-7 along with pharmaceutically acceptable excipients.

10. The pharmaceutical composition as claimed in claim 9, wherein the pharmaceutical composition is used for the preparation of a medicament.

11. Medicament as claimed in claim 10 for the treatment of hyperuricemia, gout and/or for reducing serum uric acid levels.

12. The pharmaceutical composition as claimed in claim 9, for the treatment of a metabolic disorder or disease, wherein the metabolic disorder or disease is associated with increased or elevated levels of uric acid.

13. The pharmaceutical composition as claimed in claim 12, for the treatment of metabolic disorder or disease wherein the metabolic disease or disorder includes hyperuricemia, gout, Lesch-Nyan syndrome, Cardiovascular disease, Type 2 diabetes, Metabolic syndrome and/or Uric acid stone formation, Tumor lysis syndrome, osteogenesis imperfect.

## Patentansprüche

1. Uricase-Stammmutanten, ausgewählt aus Hinterlegungen mit den Zugangsnummern MTCC No. 5797 und MTCC No. 5798.

2. Uricase-Stammmutanten nach Anspruch 1, die Proteine mit den SEQ ID No.: 2 oder SEQ ID No.: 3 exprimieren, wobei die Proteine von Nukleotiden mit der SEQ ID No.:5 bzw. der SEQ ID No.: 6 codiert werden.

3. Rekombinanter Vektor, der fähig ist, Proteine mit den Sequenzen SEQ ID No.: 2 und SEQ ID No.: 3 zu exprimieren.

4. Rekombinanter Vektor nach Anspruch 3, umfassend Nukleotid mit den Sequenzen SEQ ID No.: 5 und SEQ ID No.: 6.

5. Verfahren zur Herstellung von Uricase-Stammmutanten mit den Zugangsnummern MTCC No. 5797 & MTCC No. 5798, wobei das Verfahren die folgenden Schritte umfasst:
(a) Isolieren eines Nukleotids von Interesse mit der SEQ ID No.: 4 unter Verwendung von Primern;
(b) Erzeugen von Mutation in SEQ ID No.: 4 an einer Position entsprechend Position 62 und/oder 167 in der Proteinsequenz, um zu rekombinanten Sequenzen zu gelangen;
(c) Klonieren der in Schritt (b) erhaltenen rekombinanten Nukleotide in ein Vektorkonstrukt, um zu einem rekombinanten Vektor zu gelangen;
(d) Transformieren des in Schritt (d) erhaltenen rekombinanten Vektors in einen geeigneten Wirt für die Expression der rekombinanten Sequenzen; und
(e) Erhalten der Uricase-Stammmutanten.

6. Verfahren nach Anspruch 5, wobei die rekombinanten Nukleotide SEQ ID No.: 5 und SEQ ID No.: 6 aufweisen und die rekombinanten Nukleotide für rekombinante Proteine mit der SEQ ID No.: 2 und SEQ ID No.: 3 codieren.

7. Verfahren nach Anspruch 6, wobei es sich bei den rekombinanten Proteinen um Uricaseproteine handelt.

8. Verfahren nach Anspruch 5, wobei die Primer den SEQ ID No.: 9, SEQ ID No.: 10, SEQ ID No.: 11 und SEQ ID No.: 12 entsprechen, wobei SEQ ID No.: 9 und SEQ ID No.: 10 Vorwärts- und Rückwärts-Primer für Nukleotid mit der SEQ ID No.: 5 sind und SEQ ID No.: 11 und SEQ ID No.: 12 Vorwärts- und Rückwärts-Primer für Nukleotid mit der SEQ ID No.: 6 sind.

9. Pharmazeutische Zusammensetzung, umfassend eine Uricase nach den Ansprüchen 1-7 zusammen mit pharmazeutisch unbedenklichen Grundstoffen.

10. Pharmazeutische Zusammensetzung nach Anspruch 9, wobei die pharmazeutische Zusammensetzung für die Herstellung eines Arzneimittels verwendet wird.

11. Arzneimittel nach Anspruch 10 für die Behandlung von Hyperuricämie, Gicht und/oder zum Vermindern der Serumharnsäurespiegel.

12. Pharmazeutische Zusammensetzung nach Anspruch 9 für die Behandlung einer metabolischen Störung oder Krankheit, wobei die metabolische Störung oder Krankheit mit verstärkten oder erhöhten Harnsäurespiegeln assoziiert ist.

13. Pharmazeutische Zusammensetzung nach Anspruch 12 für die Behandlung einer metabolischen Störung oder Krankheit, wobei die metabolische Störung oder Krankheit Folgendes beinhaltet: Hyperuricämie, Gicht, Lesch-Nyan-Syndrom, Herz-Kreislauf-Krankheit, Typ-2-Diabetes, metabolisches Syndrom und/oder Harnsäuresteinbildung, Tumorlyse-Syndrom, Osteogenesis imperfecta.

## Revendications

1. Souches mutantes d'uricase sélectionnées parmi les dépôts ayant les numéros d'accession MTCC n° 5797 et MTCC n° 5798.

2. Souches mutantes d'uricase selon la revendication 1 exprimant des protéines avec SEQ ID n° : 2 ou SEQ ID n° :3, les protéines étant codées par des nucléotides ayant SEQ ID n° : 5 ou SEQ ID n° : 6.

3. Vecteur recombinant capable d'exprimer des protéines ayant les séquences SEQ ID n° 2 et SEQ ID n° 3.

4. Vecteur recombinant selon la revendication 3, comprenant un nucléotide ayant les séquences SEQ ID n° 5 et SEQ ID n° 6.

5. Procédé de préparation de souches mutantes d'uricase ayant les numéros d'accession MTCC n° 5797 et MTCC No.5798, ledit procédé comprenant les étapes de :
(a) isolement d'un nucléotide d'intérêt ayant SEQ ID n° 4 au moyen d'amorces ;
(b) création d'une mutation dans SEQ ID n° 4 à la position correspondant à la position 62 et/ou 167 dont la séquence de protéines pour obtenir des séquences recombinantes ;
(c) clonage des nucléotides recombinants obtenus dans l'étape (b) dans une construction de vecteur pour obtenir un vecteur recombinant ;
(d) transformation du vecteur recombinant obtenu dans l'étape (d) dans un hôte adapté pour l'expression des séquences recombinantes ; et
(e) obtention des souches mutantes d'uricase.

6. Procédé selon la revendication 5, dans lequel les nucléotides recombinants ont les séquences SEQ ID n° : 5 et SEQ ID n° : 6 et lesdits nucléotides recombinants codent pour des protéines recombinantes ayant SEQ ID n° : 2 et SEQ ID n° : 3.

7. Procédé selon la revendication 6, dans lequel les protéines recombinantes sont l'uricase.

8. Procédé selon la revendication 5, dans lequel les amorces sont représentées par SEQ ID n° : 9, SEQ ID n° : 10, SEQ ID n° : 11 et SEQ ID n° : 12, où SEQ ID n° : 9 et SEQ ID n° : 10 sont des amorces sens et antisens pour un nucléotide avec SEQ ID n° : 5 et SEQ ID n° : 11 et SEQ ID n° : 12 sont des amorces sens et antisens pour un nucléotide avec SEQ ID n° : 6.

9. Composition pharmaceutique comprenant une uricase selon les revendications 1 à 7 avec des excipients pharmaceutiquement acceptables.

10. Composition pharmaceutique selon la revendication 9, la composition pharmaceutique étant utilisée pour la préparation d'un médicament.

11. Médicament selon la revendication 10 pour le traitement de l'hyperuricémie, la goutte et/ou pour abaisser les taux sériques d'acide urique.

12. Composition pharmaceutique selon la revendication 9, pour le traitement d'un trouble ou une maladie métabolique, le trouble ou la maladie métabolique étant associé à des taux augmentés ou élevés d'acide urique.

13. Composition pharmaceutique selon la revendication 12, pour le traitement d'un trouble ou une maladie métabolique, la maladie ou le trouble métabolique comprenant l'hyperuricémie, la goutte, le syndrome de Lesch-Nyan, une maladie cardiovasculaire, le diabète de type 2, le syndrome métabolique et/ou la formation de calculs d'acide urique, le syndrome de lyse tumorale, l'ostéogenèse imparfaite.
